# EUROPÄISCHE PATENTANMELDUNG

(11) **EP 2 103 604 A1**
(43) Veröffentlichungstag der Anmeldung: **23.09.2009**
(21) Anmeldenummer: 08102653.6
(22) Anmeldetag: 17.03.2008
(51) Int. Cl.: C07D 301/12

(54) **Verfahren zur Herstellung von Epichlorhydrin**

(71) Anmelder: Evonik Degussa GmbH, 45128 Essen (DE)
(72) Erfinder: Hofen, Willi, 63517 Rodenbach (DE); Brasse, Claudia, 63452 Hanau (DE); Franke, Robert, 45772 Marl (DE); Katzer, Robert, 60594 Frankfurt am Main (DE)

(57) **Zusammenfassung**

Verfahren zur Herstellung von Epichlorhydrin, bei dem in einer ersten Reaktionsstufe ein Chlorpropane enthaltendes Allylchlorid im Überschuss mit Wasserstoffperoxid umgesetzt wird. Das nicht umgesetzte Allylchlorid wird abgetrennt und in die Reaktion zurückgeführt, wobei ein Teil des abgetrennten Allylchlorids einer zweiten Reaktionsstufe zugeführt und mit Wasserstoffperoxid umgesetzt wird, wobei die Wasserstoffperoxidmenge in der zweiten Reaktionsstufe so gewählt ist, dass das Allylchlorid weitgehend umgesetzt wird. Aus der Reaktionsmischung der zweiten Reaktionsstufe werden die Chlorpropane durch Destillation abgetrennt und aus dem Verfahren entnommen.

## Beschreibung

Die Erfindung betrifft ein Verfahren zur Herstellung von Epichlorhydrin durch Umsetzung von Allylchlorid mit Wasserstoffperoxid.

Epichlorhydrin (Chlormethyloxiran) ist ein wichtiges chemisches Zwischenprodukt, das z. B. zur Herstellung von Harzen verwendet wird.

Ein zur Herstellung von Epichlorhydrin geeignetes Verfahren ist die aus EP-A 0 100 119 bekannte Umsetzung von Allylchlorid mit Wasserstoffperoxid in Gegenwart eines titanhaltigen Zeolithkatalysators. Um bei dieser Reaktion eine hohe Selektivität für Epichlorhydrin zu erhalten, muss Allylchlorid in stöchiometrischem Überschuss zu Wasserstoffperoxid eingesetzt werden, wie beispielsweise aus WO 2004/043941 bekannt ist. Nicht umgesetztes Allylchlorid kann durch Destillation abgetrennt und in die Epoxidierungsreaktion zurückgeführt werden, wie z. B. aus WO 02/00634 oder WO 02/14298 bekannt ist.

Technisches Allylchlorid ist in der Regel mit 1-Chlorpropan und/oder 2-Chlorpropan verunreinigt. Beide Verunreinigungen haben ähnlich Siedepunkte wie Allylchlorid:
Allylchlorid: 45°C
1-Chlorpropan: 47°C
2-Chlorpropan: 36°C

Die beiden Chlorpropane lassen sich von Allylchlorid deshalb nur mit großem Aufwand destillativ abtrennen. Wenn für die Umsetzung von Allylchlorid mit Wasserstoffperoxid ein technisches, mit Chlorpropanen verunreinigtes Allylchlorid eingesetzt wird und nicht umgesetztes Allylchlorid durch Destillation abgetrennt und in die Epoxidierungsreaktion zurückgeführt wird, kommt es demnach zu einer Anreicherung von Chlorpropanen im Verfahren.

Aus WO 02/00634 und WO 02/14298 ist bekannt, dass sich eine Anreicherung von Verunreinigungen im zurückgeführten, nicht umgesetzten Olefin auf unerwünscht hohe Konzentrationen durch die Ausschleusung eines Teils des zurückgeführten Olefins aus dem Verfahren verhindern lässt. Der ausgeschleuste Teilstrom enthält allerdings einen hohen Anteil an Olefin, der durch die Ausschleusung verloren geht.

Es besteht deshalb ein Bedarf nach einem Verfahren zur Herstellung von Epichlorhydrin durch Umsetzung eines Chlorpropane enthaltenden Allylchlorids mit einer hohen Selektivität für Epichlorhydrin und einer gegenüber den bekannten Verfahren verbesserten Umsetzung des eingesetzten Allylchlorids.

Diese Aufgabe wird gelöst durch ein erfindungsgemäßes Verfahren, bei dem in einer ersten Reaktionsstufe ein Chlorpropane enthaltendes Allylchlorid im Überschuss mit Wasserstoffperoxid umgesetzt wird. Das nicht umgesetzte Allylchlorid wird abgetrennt und in die Reaktion zurückgeführt wird, wobei ein Teil des abgetrennten Allylchlorids einer zweiten Reaktionsstufe zugeführt und mit Wasserstoffperoxid umgesetzt wird, wobei die Wasserstoffperoxidmenge in der zweiten Reaktionsstufe so gewählt ist, dass das Allylchlorid weitgehend und vorzugsweise praktisch vollständig umgesetzt wird. Aus der Reaktionsmischung der zweiten Reaktionsstufe lassen sich die Chlorpropane dann ohne Verluste an Allylchlorid durch Destillation abtrennen. Da in dem erfindungsgemäßen Verfahren der größte Teil des Allylchlorids bei einem Überschuss an Allylchlorid umgesetzt wird und nur ein kleiner Teil des Allylchlorids bei einem geringen Überschuss oder einem Unterschuss an Allylchlorid reagiert, bleibt die durch einen Überschuss an Allylchlorid bewirkte hohe Selektivität für Epichlorhydrin erhalten.

Gegenstand der Erfindung ist deshalb ein Verfahren zur Herstellung von Epichlorohydrin, bei dem
a) in einer ersten Reaktionsstufe Allylchlorid und Wasserstoffperoxid in Gegenwart eines titanhaltigen Zeolithkatalysators in einem molaren Verhältnis von Allylchlorid zu Wasserstoffperoxid im Bereich von 1,5:1 bis 5:1 umgesetzt werden und wobei das eingesetzte Allylchlorid 1-Chlorpropan und/oder 2-Chlorpropan enthält,
b) die in der ersten Reaktionsstufe gebildete Reaktionsmischung in einer Destillation aufgetrennt wird in eine Mischung (A), die nicht umgesetztes Allylchlorid, sowie 1-Chlorpropan und/oder 2-Chlorpropan enthält und eine Mischung (B), die Epichlorhydrin enthält,
c) die Mischung (A) aufgeteilt wird in eine Mischung (A1), die in die erste Reaktionsstufe zurückgeführt wird und eine Mischung (A2),
d) die Mischung (A2) in einer zweiten Reaktionsstufe mit Wasserstoffperoxid in Gegenwart eines titanhaltigen Zeolithkatalysators in einem molaren Verhältnis von Allylchlorid zu Wasserstoffperoxid im Bereich von 0,5:1 bis 1,25:1 umgesetzt wird,
e) die in der zweiten Reaktionsstufe gebildete Reaktionsmischung in einer Destillation aufgetrennt wird in eine Mischung (C), die 1-Chlorpropan und/oder 2-Chlorpropan enthält und eine Mischung (D), die Epichlorhydrin enthält und
f) die Mischung (C) aus dem Verfahren entnommen wird.

Bei dem erfindungsgemäßen Verfahren werden Allylchlorid und Wasserstoffperoxid in Gegenwart eines titanhaltigen Zeolithkatalysators zu Epichlorhydrin umgesetzt. Das eingesetzte Allylchlorid enthält dabei 1-Chlorpropan und/oder 2-Chlorpropan. Für das erfindungsgemäße Verfahren können deshalb technische Qualitäten von Allylchlorid verwendet werden, die 1-Chlorpropan und/oder 2-Chlorpropan als Nebenprodukte der technischen Herstellung von Allylchlorid enthalten. Der Gehalt an 1-Chlorpropan und 2-Chlorpropan im eingesetzten Allylchlorid liegt vorzugsweise im Bereich von 0,01 bis 2 Gew.-%, besonders bevorzut im Bereich von 0,05 bis 0,8 Gew.-%.

Wasserstoffperoxid kann als wässrige Lösung eingesetzt werden, die vorzugsweise einen Gehalt an Wasserstoffperoxid im Bereich von 1 bis 90 Gew.-%, besonders bevorzugt von 10 bis 80 Gew.-% und insbesondere von 30 bis 70 Gew.-%, aufweist. Wasserstoffperoxid kann als handelsübliche, stabilisierte Lösung eingesetzt werden. Ebenso geeignet ist nach dem Anthrachinonverfahren hergestelltes, nicht stabilisiertes Wasserstoffperoxid, das ohne weitere Reinigung eingesetzt werden kann. Vorzugsweise wird ein aus WO 2004/028962 bekanntes Wasserstoffperoxid verwendet, dass weniger als 50 ppm Alkalimetalle und Erdalkalimetalle, weniger als 50 ppm Basen mit einem pK_{B} von weniger als 4,5 und mindestens 100 ppm Anionen, jeweils bezogen auf das Gewicht an Wasserstoffperoxid, enthält.

In einer weiteren bevorzugten Ausführungsform wird eine Lösung von Wasserstoffperoxid in Methanol verwendet, die vorzugsweise durch Umsetzung von Wasserstoff und Sauerstoff an einem Palladiumkatalysator in Methanol hergestellt wurde. Besonders bevorzugt wird eine Wasserstoffperoxidlösung in Methanol gemäß Anspruch 9 von WO 2006/108784 verwendet, die 2 bis 15 Gew.-% Wasserstoffperoxid, 0,5 bis 20 Gew.-% Wasser, 60 bis 95 Gew.-% Methanol, 10⁻⁶ bis 10⁻² mol/l Bromid, sowie 10⁻⁶ bis 0,1 mol/l Dimethylsulfat und/oder Monomethylsulfat enthält.

Als titanhaltiger Zeolithkatalysator können alle aus dem Stand der Technik bekannten titanhaltigen Zeolithe verwendet werden, die eine katalytische Aktivität für die Umsetzung von Olefinen mit Wassesrtoffperoxid aufweisen. Vorzugsweise wird als titanhaltiger Zeolithkatalysator ein Titansilicalit mit MFI- oder MEL-Kristallstruktur eingesetzt. Besonders bevorzugt werden Titansilicalite der Zusammensetzung (TiO₂)ₓ(SiO₂)₁₋ₓ verwendet, wobei x im Bereich von 0,001 bis 0,05 liegt. Am meisten bevorzugt sind Titansilicalite, die nach dem Verfahren gemäß WO 01/64581 oder dem Verfahren gemäß WO 01/64582 hergestellt wurden.

Der titanhaltige Zeolithkatalysator kann im erfindungsgemäßen Verfahren in Form eines Suspensionskatalysators verwendet werden. In diesem Fall wird die Umsetzung bevorzugt so durchgeführt, dass der in der Reaktionsmischung suspendierte Katalysator in der ersten Reaktionsstufe zurückgehalten wird, beispielsweise durch Filtration oder durch Sedimentation, so dass die Reaktionsmischung, die in Schritt b) in einer Destillation aufgetrennt wird, keinen Katalysator enthält.

Vorzugsweise wird der titanhaltige Zeolithkatalysator im erfindungsgemäßen Verfahren jedoch in Form eines Festbettkatalysators verwendet. Besonders geeignet sind durch Extrusion verformte Festbettkatalysatoren in Form von Extrudaten mit einem Durchmesser von 1 bis 5 mm, die vorzugsweise ein Bindemittel in einer Menge von 1 bis 99 Gew.-%, besonders bevorzugt 1 bis 40 Gew.-%, bezogen auf den titanhaltigen Zeolith enthalten. Geeignet sind dabei alle Bindemittel, die unter den Reaktionsbedingungen weder mit dem eingesetzten Wasserstoffperoxid noch mit dem gebildeten Epichlorhydrin reagieren. Besonders geeignete Bindemittel sind Kieselsäuren. Besonders bevorzugt sind Festbettkatalysatoren, bei denen zur Extrusion eine pyrogene Kieselsäure, ein kolloidales Kieselsol oder ein Tetraalkylorthosilikat oder eine Kombination von zwei dieser Komponenten als Vorläufer für das Bindemittel eingesetzt wurden. Ebenfalls besonders bevorzugt sind Festbettkatalysatoren, die nach dem aus WO 01/72419 bekannten Verfahren durch Verformen einer Formmasse hergestellt wurden, die einen Plateauwert der Curdkurve im Bereich von 20 bis 90 mm aufweist.

Bei dem erfindungsgemäßen Verfahren werden in Schritt a) in der ersten Reaktionsstufe Allylchlorid und Wasserstoffperoxid in einem molaren Verhältnis von Allylchlorid zu Wasserstoffperoxid im Bereich von 1,5:1 bis 5:1 umgesetzt. Vorzugsweise beträgt das molare Verhältnis von Allylchlorid zu Wasserstoffperoxid 2:1 bis 4:1. Bei einem kleineren molaren Verhältnis nimmt die Selektivität für Epichlorhydrin in der ersten Reaktionsstufe ab. Höhere molare Verhältnisse haben den Nachteil, dass große Mengen an nicht umgesetztem Allylchlorid mit entsprechendem Energieaufwand abgetrennt und zurückgeführt werden müssen.

In Schritt d) des erfindungsgemäßen Verfahrens werden in der zweiten Reaktionsstufe Allylchlorid und Wasserstoffperoxid in einem molaren Verhältnis von Allylchlorid zu Wasserstoffperoxid im Bereich von 0,5:1 bis 1,25:1 umgesetzt. Vorzugsweise beträgt das molare Verhältnis von Allylchlorid zu Wasserstoffperoxid 0,8:1 bis 1,15:1. Die Verwendung eines molaren Verhältnisses in diesen Bereichen ermöglicht es, in der zweiten Reaktionsstufe Allylchlorid ganz oder zum größten Teil umzusetzen, so dass die in Schritt e) erhaltene und in Schritt f) aus dem Verfahren entnommene Mischung (C) nur einen geringen Anteil des eingesetzten Allylchlorids enthält.

Die Umsetzung von Allylchlorid und Wasserstoffperoxid erfolgt in den Schritten a) und d) vorzugsweise in Gegenwart eines Lösungsmittels. Besonders geeignet sind Lösungsmittel, die unter den Reaktionsbedingungen Allylchlorid und Wasserstoffperoxid lösen und nicht oder nur in einem geringen Maß mit Wasserstoffperoxid oder Epichlorhydrin reagieren. Als Lösungsmittel eignen sich beispielsweise Alkohole, wie Methanol, Ethanol oder tert-Butanol; Glykole, wie beispielsweise Ethylenglykol, 1,2-Propandiol oder 1,3-Propandiol; cyclische Ether, wie beispielsweise Tetrahydrofuran oder Dioxan; Glykolether, wie beispielsweise Ethylenglykolmonomethylether, Ethylenglykolmonoethylether, Ethylenglykolmonobutylether oder Propylenglykolmonomethylether; sowie Ketone, wie beispielsweise Aceton oder 2-Butanon. Bevorzugte Lösungsmittel sind aliphatische Alkohole mit 1 bis 4 Kohlenstoffatomen. Besonders bevorzugt wird Methanol als Lösungsmittel verwendet. Der Anteil an Lösungsmittel an der Reaktionsmischung beträgt in der ersten Reaktionsstufe vorzugsweise 10 bis 95 Gew.-%, besonders bevorzugt 30 bis 80 Gew.-% und in der zweiten Reaktionsstufe vorzugsweise 10 bis 95 Gew.-%, besonders bevorzugt 30 bis 80 Gew.-%.

Die Umsetzung von Allylchlorid und Wasserstoffperoxid erfolgt in der ersten Reaktionsstufe vorzugsweise bei einer Temperatur im Bereich von 0°C bis 100°C, besonders bevorzugt 30°C bis 65°C und in der zweiten Reaktionsstufe vorzugsweise bei einer Temperatur im Bereich von 0°C bis 100°C, besonders bevorzugt 30°C bis 65°C. Der Druck in den Reaktionsstufen kann in weiten Grenzen frei gewählt werden und wird vorzugsweise so hoch gewählt, dass der Siedepunkt von Allylchlorid bei dem verwendeten Druck gleich oder höher ist als die verwendete Reaktionstemperatur.

Die Reaktionsbedingungen werden in der ersten Reaktionsstufe vorzugsweise so gewählt, dass ein Umsatz von Wasserstoffperoxid im Bereich von 50% bis 100%, vorzugsweise von 80% bis 99,8%, erreicht wird. In der zweiten Reaktionsstufe werden die Reaktionsbedingungen vorzugsweise so gewählt, dass von den Komponenten Allylchlorid und Wasserstoffperoxid die im molaren Unterschuss eingesetzte Komponente zu 70% bis 100%, vorzugsweise zu 90% bis 99%, umgesetzt wird.

Zur Umsetzung von Allylchlorid und Wasserstoffperoxid in den Schritten a) und d) können alle für die Durchführung von Flüssigphasenreaktionen geeigneten Reaktoren verwendet werden. Die Umsetzung kann dabei sowohl satzweise als auch kontinuierlich erfolgen, wobei eine kontinuierliche Umsetzung bevorzugt ist.

Vorzugsweise erfolgt die Umsetzung in den Schritten a) und d) kontinuierlich in einem Festbettreaktor, wobei eine Mischung, die Wasserstoffperoxid, gegebenenfalls Lösungsmittel sowie Allylchlorid bzw. die Mischung (A2) enthält, über ein Festbett des titanhaltigen Zeolithkatalysators geleitet wird. Als Festbettreaktor wird vorzugsweise ein von außen gekühlter Rohrreaktor, insbesondere ein Rohrbündelreaktor verwendet. Der Festbettreaktor kann sowohl in Aufwärtsströmung als auch in Abwärtsströmung betrieben werden, wobei ein Betrieb mit Abwärtsströmung im Rieselbettzustand bevorzugt wird.

Sowohl in Schritt a) als auch in Schritt d) kann die Umsetzung in zwei oder mehr in Reihe geschalteten Reaktoren durchgeführt werden. Vorzugsweise werden in Schritt a) zwei in Reihe geschaltete Reaktoren verwendet. Sowohl in Schritt a) als auch in Schritt d) können zwei oder mehr parallel angeordnete Reaktoren verwendet werden, so dass ein Reaktor für eine Regenerierung des Katalysators außer Betrieb genommen werden kann und die Umsetzung in einem parallel geschalteten Reaktor fortgesetzt werden kann.

Die in der ersten Reaktionsstufe gebildete Reaktionsmischung wird in Schritt b) des erfindungsgemäßen Verfahrens in einer Destillation aufgetrennt wird in eine Mischung (A), die nicht umgesetztes Allylchlorid, sowie 1-Chlorpropan und/oder 2-Chlorpropan enthält und eine Mischung (B), die Epichlorhydrin enthält. Die Destillation wird vorzugsweise als kontinuierliche Rektifikation durchgeführt, wobei einer Rektifikationskolonne in einem mittleren Abschnitt die in der ersten Reaktionsstufe gebildete Reaktionsmischung zugeführt wird, am Kopf der Kolonne die Mischung (A) entnommen wird und aus dem Sumpf der Kolonne die Mischung (B) entnommen wird. Vorzugsweise wird eine Rektifikationskolonne mit 10 bis 50 theoretischen Trennstufen verwendet. Die Rektifikation erfolgt vorzugsweise bei einem Druck am Kolonnenkopf im Bereich von 0,2 bis 3 bar und vorzugsweise bei einem Rücklaufverhältnis von 0,5 bis 5.

Die Destillation wird vorzugsweise so betrieben, dass die resultierende Mischung (A) mehr als 95% des in der zugeführten Reaktionsmischung enthaltenen Allylchlorids enthält und die resultierende Mischung (B) mehr als 95% des in der zugeführten Reaktionsmischung enthaltenen Epichlorhydrins enthält.

In Schritt c) des erfindungsgemäßen Verfahrens wird die Mischung (A) aufgeteilt in eine Mischung (A1), die in die erste Reaktionsstufe zurückgeführt wird und eine Mischung (A2), die der zweiten Reaktionsstufe zugeführt wird.

Vorzugsweise wird die Mischung (A) so aufgeteilt, dass 50% bis 98%, besonders bevorzugt 70% bis 95% des in der Mischung (A) enthaltenen Allylchlorids mit der Mischung (A1) in die erste Reaktionsstufe zurückgeführt wird.

In einer bevorzugten Ausführungsform wird die Mischung (A) durch eine Destillation aufgetrennt, so dass die in Mischung (A) enthaltenen Chlorpropane in der Mischung (A2) angereichert werden.

In Schritt e) des erfindungsgemäßen Verfahrens wird die in der zweiten Reaktionsstufe gebildete Reaktionsmischung in einer Destillation aufgetrennt in eine Mischung (C), die 1-Chlorpropan und/oder 2-Chlorpropan enthält und eine Mischung (D), die Epichlorhydrin enthält. Die Destillation wird vorzugsweise als kontinuierliche Rektifikation durchgeführt, wobei einer Rektifikationskolonne in einem mittleren Abschnitt die in der zweiten Reaktionsstufe gebildete Reaktionsmischung zugeführt wird, am Kopf der Kolonne die Mischung (C) entnommen wird und aus dem Sumpf der Kolonne die Mischung (D) entnommen wird. Vorzugsweise wird eine Rektifikationskolonne mit 10 bis 50 theoretischen Trennstufen verwendet. Die Rektifikation erfolgt vorzugsweise bei einem Druck am Kolonnenkopf im Bereich von 0,5 bis 3 bar und vorzugsweise bei einem Rücklaufverhältnis von 0,5 bis 5.

Die Destillation wird vorzugsweise so betrieben, dass die resultierende Mischung (C) mehr als 90% der in der zugeführten Reaktionsmischung enthaltenen Chlorpropane enthält und die resultierende Mischung (D) mehr als 95% des in der zugeführten Reaktionsmischung enthaltenen Epichlorhydrins enthält.

In einer bevorzugten Ausführungsform wird die in Schritt e) des erfindungsgemäßen Verfahrens erhaltene Mischung (D) in die erste Reaktionsstufe zurückgeführt. Diese Ausführungsform ist besonders vorteilhaft, wenn in Schritt d) Wasserstoffperoxid in molarem Überschuss eingesetzt wird und die Mischung (D) noch nicht umgesetztes Wasserstoffperoxid enthält. Durch die Rückführung in die erste Reaktionsstufe kann dieses nicht umgesetzte Wasserstoffperoxid noch zur Epoxidierung von weiterem Allylchlorid genutzt werden. In einer besonders bevorzugten Ausführungsform wird die erste Reaktionsstufe in zwei in Reihe geschalteten Reaktoren durchgeführt und die Mischung (D) in den zweiten Reaktor zurückgeführt.

In einer weiteren Ausführungsform erfolgen die Schritte d) und e) des erfindungsgemäßen Verfahrens gleichzeitig in Form einer Reaktivdestillation. Bei dieser Ausführungsform wird der titanhaltige Zeolithkatalysator der zweiten Reaktionsstufe in einem Reaktionsabschnitt einer Rektifikationskolonne angeordnet, die Mischung (A2) der Kolonne an einem Punkt unterhalb des Reaktionsabschnitts zugeführt und Wasserstoffperoxid an einem Punkt oberhalb des Reaktionsabschnitts zugeführt. Am Kopf der Kolonne wird die Mischung (C) entnommen und aus dem Sumpf der Kolonne wird die Mischung (D) entnommen.

## Patentansprüche

1. Verfahren zur Herstellung von Epichlorohydrin, wobei
a) in einer ersten Reaktionsstufe Allylchlorid und Wasserstoffperoxid in Gegenwart eines titanhaltigen Zeolithkatalysators in einem molaren Verhältnis von Allylchlorid zu Wasserstoffperoxid im Bereich von 1,5:1 bis 5:1 umgesetzt werden und wobei das eingesetzte Allylchlorid 1-Chlorpropan und/oder 2-Chlorpropan enthält,
b) die in der ersten Reaktionsstufe gebildete Reaktionsmischung in einer Destillation aufgetrennt wird in eine Mischung (A), die nicht umgesetztes Allylchlorid, sowie 1-Chlorpropan und/oder 2-Chlorpropan enthält und eine Mischung (B), die Epichlorhydrin enthält,
c) die Mischung (A) aufgeteilt wird in eine Mischung (A1), die in die erste Reaktionsstufe zurückgeführt wird und eine Mischung (A2),
d) die Mischung (A2) in einer zweiten Reaktionsstufe mit Wasserstoffperoxid in Gegenwart eines titanhaltigen Zeolithkatalysators in einem molaren Verhältnis von Allylchlorid zu Wasserstoffperoxid im Bereich von 0,5:1 bis 1,25:1 umgesetzt wird,
e) die in der zweiten Reaktionsstufe gebildete Reaktionsmischung in einer Destillation aufgetrennt wird in eine Mischung (C), die 1-Chlorpropan und/oder 2-Chlorpropan enthält und eine Mischung (D), die Epichlorhydrin enthält und
f) die Mischung (C) aus dem Verfahren entnommen wird.

2. Verfahren nach Anspruch 1,
**dadurch gekennzeichnet,**
**dass** die Umsetzung von Allylchlorid und Wasserstoffperoxid in den Schritten a) und d) in Gegenwart eines Lösungsmittels erfolgt.

3. Verfahren nach Anspruch 2,
**dadurch gekennzeichnet,**
**dass** das Lösungsmittel Methanol ist.

4. Verfahren nach Anspruch 3,
**dadurch gekennzeichnet,**
**dass** Wasserstoffperoxid in Form einer Lösung von Wasserstoffperoxid in Methanol eingesetzt wird.

5. Verfahren nach einem der vorangehenden Ansprüche,
**dadurch gekennzeichnet,**
**dass** die Aufteilung der Mischung (A) in Schritt c) durch eine Destillation erfolgt, bei der die in Mischung (A) enthaltenen Chlorpropane in der Mischung (A2) angereichert werden.

6. Verfahren nach einem der vorangehenden Ansprüche,
**dadurch gekennzeichnet,**
**dass** die Mischung (D) in die erste Reaktionsstufe zurückgeführt wird.

7. Verfahren nach Anspruch 6,
**dadurch gekennzeichnet,**
**dass** die erste Reaktionsstufe in zwei in Reihe geschalteten Reaktoren durchgeführt wird und die Mischung (D) in den zweiten Reaktor zurückgeführt wird.

8. Verfahren nach einem der vorangehenden Ansprüche,
**dadurch gekennzeichnet,**
**dass** die Schritte d) und e) gleichzeitig in einer Reaktivdestillation erfolgen.
